(19) Europäisches Patentamt
European Patent Office
Office européen des brevets

(11) EP 1 911 760 A1

(12) **EUROPEAN PATENT APPLICATION**
published in accordance with Art. 153(4) EPC

(43) Date of publication:
**16.04.2008 Bulletin 2008/16**

(21) Application number: **06780819.6**

(22) Date of filing: **29.06.2006**

(51) Int Cl.:
*C07D 473/00* (2006.01) *A61K 31/519* (2006.01)
*A61K 31/52* (2006.01) *A61K 31/5377* (2006.01)
*A61K 31/541* (2006.01) *A61P 19/06* (2006.01)
*A61P 43/00* (2006.01) *C07D 498/04* (2006.01)
*C07D 513/04* (2006.01)

(86) International application number:
**PCT/JP2006/313443**

(87) International publication number:
**WO 2007/004688 (11.01.2007 Gazette 2007/02)**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK RS**

(30) Priority: **01.07.2005 JP 2005193964**

(71) Applicant: **Nippon Chemiphar Co., Ltd.**
**Chiyoda-ku**
**Tokyo 1010032 (JP)**

(72) Inventors:
- **NISHINO, Takeshi**
  **Kanagawa, 2360052 (JP)**
- **YOSHIDA, Shinichi**
  **Chiba, 2730031 (JP)**
- **TENDO, Atsushi**
  **Saitama, 3440005 (JP)**
- **YAMAKAWA, Tomio**
  **Chiba, 2770884 (JP)**
- **KOBAYASHI, Tadashi**
  **Tokyo, 1850014 (JP)**
- **SHINOHARA, Yoriko**
  **Chiba-shi,**
  **Chiba, 2620032 (JP)**

(74) Representative: **Albrecht, Thomas et al**
**Kraus & Weisert**
**Thomas-Wimmer-Ring 15**
**80539 München (DE)**

# (54) XANTHINE OXIDASE INHIBITOR

(57) A compound represented by the following formula (I) is used as a xanthine oxidase inhibitor:

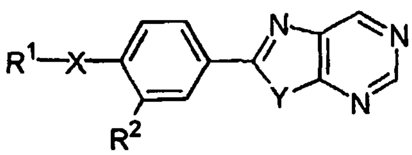

(I)

in which $R^1$ is phenyl or pyridyl each optionally having, as a substituent, $C_{1-8}$ alkyl, $C_{1-8}$ haloalkyl, $C_{1-8}$ alkoxy, carboxyl, halogeno, hydroxyl, nitro, cyano, amino, etc.; $R^2$ is cyano, nitro, etc.; X is oxygen, sulfur, etc.; and Y is sulfur, NH, etc.

EP 1 911 760 A1

## Description

[FIELD OF THE INVENTION]

The present invention relates to a xanthine oxidase inhibitor.

[BACKGROUND OF THE INVENTION]

The hyperuricemia causes gout and renal insufficiency and further is considered to be a factor causing coronary disease. Furthermore, the hyperuricemia is suggested to closely relate to development of diseases of adult people such as hypertension. Therefore, treatment of the hyperuricemia can be effective not only for treating gout but also for preventing various diseases relating to daily nutrition and developing in the course of advancement of age.

At the present time, the hyperuricemia is treated using an inhibitor for inhibiting production of uremic acid such as allopurinol and an accelerator for uricotelism such as benzbromalone. However, the allopurinol is well known to cause side effects such as lesion, hepatopathy, and myelogenetic troubles. The allopurinol and its metabolic product (oxypurinol) are excreted from kidney. However, if the excretion of uric acid decreases, the excretion of these compounds also decreases and the their concentrations in blood increase. Therefore, the chance of causing side effects increases.

It is reported that benzbromalone also causes hepatopathy. Accordingly, it is desired to develop new pharmaceuticals so that the practitioners can select most appropriate pharmaceuticals.

Recently, the below-mentioned xanthine oxidase inhibitors having no purine nucleus such as TMX-67 (Teijin Corporation, Patent Publication 1: WO 92/09279), Y-700 (Mitsubishi Wellpharma Corporation, Patent Publication 2: WO 98/18765), KT651 (Kotobuki Corporation, Patent Publication 3: JP-A-12-1431), and FYX-051 (Fuji Pharmaceuticals Corporation, Patent Publication 4:WO 03/064410) have been reported:

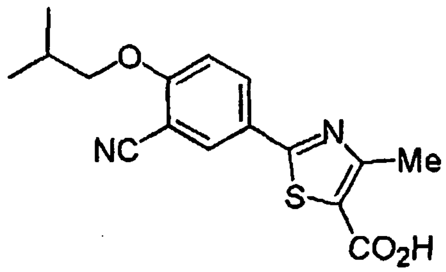

TMX-67

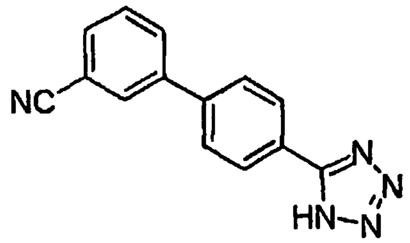

KT651

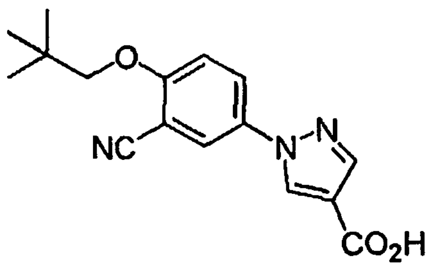

Y-700

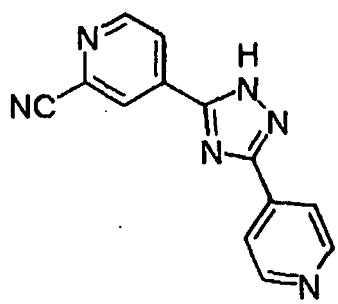

FYX-051

The present inventors made studies on bicyclic condensed hetero rings having a structure differing from the above-mentioned structures and filed a patent application (Patent Publication 5: WO 03/042185).

The inventors further have made studies and discovered that 2-(4-phenoxy-3-cyanophenyl)thiazolo[5,4-d]-pyrimidine derivatives having the below-mentioned formula (I) have a xanthine oxidase inhibiting effect and a uric acid

decrease effect. The present invention has been completed based on the discovery.

[DISCLOSURE OF THE INVENTION]

The present invention has an object to provide compounds of the below-mentioned formula (I) which have a xanthine oxidase (XOD) inhibiting effect.

The invention resides in the compounds of the following formula (I) and their salts:

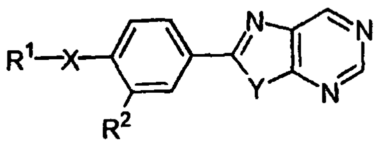

(I)

in which

$R^1$ represents an alkenyl group having 2-8 carbon atoms, or an aryl group having 6-10 carbon atoms or a hetero-aryl group which may have a substituent selected from the group and atom consisting of an alkyl group having 1-8 carbon atoms, a halogen-substituted alkyl group having 1-8 carbon atoms, an alkoxy group having 1-8 carbon atoms, a halogen-substituted alkoxy group having 1-8 carbon atoms, an alkoxycarbonyl group having 2-8 carbon atoms, formyl, carboxyl, a halogen atom, hydroxyl, nitro, cyano, amino, an aryl group having 6-10 carbon atoms, and an aryloxy group having 6-10 carbon atoms;

$R^2$ represents cyano, nitro, formyl, carboxyl, carbamoyl, or an alkoxycarbonyl group having 2-8 carbon atoms;

X represents oxygen, $-N(R^3)-$ or $-S(O)_n-$ in which $R^3$ is hydrogen, an alkyl group having 1-8 carbon atoms or the above-mentioned group described for $R^1$, or $R^3$ is, in combination with $R^1$, morpholinyl, thiomorpholinyl or piperazinyl, and n is an integer of 0 to 2; and

Y represents oxygen, sulfur or NH.

Further, the invention relates to a xanthine oxidase inhibitor containing a compound of the formula (I) or a salt thereof as an active component.

Furthermore, the invention relates to an agent for treating hyperuricemia containing a compound of the formula (I) or a salt thereof as an active component.

[Preferred Embodiments of the Invention]

The invention is further described below in detail.

Examples of the alkenyl groups having 2-8 carbon atoms for $R^1$ include allyl.

Examples of the alkyl groups having 1-8 carbon atoms which are optionally attachable substituents of the aryl group having 6-10 carbon atoms or hetero-aryl group for $R^3$ and $R^1$ include methyl, ethyl, propyl, isopropyl, butyl, isobutyl, t-butyl, and pentyl.

Examples of the halogen-substituted alkyl groups having 1-8 carbon atoms which are optionally attachable substituents for the aryl group having 6-10 carbon atoms or hetero-aryl group for $R^1$ include methyl, ethyl, propyl, isopropyl, butyl, isobutyl, t-butyl, and pentyl which have a substituent such as 1 to 3 fluorine, chlorine, or bromine atoms.

Examples of the alkoxy groups having 1-8 carbon atoms which are optionally attachable substituents for the aryl group having 6-10 carbon atoms or hetero-aryl group for $R^1$ include methoxy, ethoxy, propyloxy, isopropyloxy, butyloxy, isobutyloxy, t-butyloxy, and pentyloxy.

Examples of the halogen-substituted alkoxy groups having 1-8 carbon atoms which are optionally attachable substituents for the aryl group having 6-10 carbon atoms or hetero-aryl group for $R^1$ include methoxy, ethoxy, propyloxy, isopropyloxy, butyloxy, isobutyloxy, t-butyloxy, and pentyloxy which have a substituent such as 1 to 3 fluorine, chlorine, or bromine atoms.

Examples of the alkoxycarbonyl groups having 2-8 carbon atoms which are optionally attachable substituents for the aryl group having 6-10 carbon atoms or hetero-aryl group for $R^2$ and $R^1$ include methoxycarbonyl, ethoxycarbonyl, and propyloxycarbonyl.

Examples of the halogen atoms which are optionally attachable substituents for the aryl group having 6-10 carbon atoms or hetero-aryl group for $R^1$ include fluorine, chlorine, and bromine.

Examples of the aryl groups having 6-10 carbon atoms for $R^1$ and the aryl groups having 6-10 carbon atoms which are optionally attachable substituents for the aryl group having 6-10 carbon atoms or hetero-aryl group for $R^1$ include phenyl and naphthyl. Preferred is phenyl.

Examples of the aryloxy group having 6-10 carbon atoms which are optionally attachable substituents for the aryl group having 6-10 carbon atoms or hetero-aryl group for $R^1$ include phenyloxy and naphthyloxy. Preferred is phenyloxy.

Examples of the hetero-aryl group for $R^1$ include furyl, pyrrolyl, thienyl, piperidinyl, pyrimidinyl, pyranyl, pyridyl, thiazolyl, imidazolyl, indolyl and quinolyl.

It is preferred that n is 0.

The compound of the formula (I) can be in the form of a pharmacologically acceptable salt. For instance, a salt with an alkali metal such as sodium, potassium, or lithium can be mentioned.

Preferred compounds according to the invention are described below.

(1) The compounds having the formula (I) or salts thereof, in which $R^1$ represents a phenyl, naphthyl, furyl, pyrrolyl, thienyl, piperidinyl, pyrimidinyl, pyranyl, pyridyl, thiazolyl, imidazolyl, indolyl or quinolyl group which may have a substituent selected from the group and atom consisting of an alkyl group having 1-8 carbon atoms, a halogen-substituted alkyl group having 1-8 carbon atoms, an alkoxy group having 1-8 carbon atoms, an alkoxycarbonyl group having 2-8 carbon atoms, formyl, carboxyl, a halogen atom, hydroxyl, nitro, cyano, amino, an aryl group having 6-10 carbon atoms, and an aryloxy group having 6-10 carbon atoms.

(2) The compounds having the formula (I) or salts thereof, in which $R^1$ represents a phenyl or pyridyl group which may have a substituent selected from the group and atom consisting of an alkyl group having 1-8 carbon atoms, a halogen-substituted alkyl group having 1-8 carbon atoms, an alkoxy group having 1-8 carbon atoms, a halogen-substituted alkoxy group having 1-8 carbon atoms, an alkoxycarbonyl group having 2-8 carbon atoms, formyl, carboxyl, a halogen atom, hydroxyl, nitro, cyano, amino, an aryl group having 6-10 carbon atoms, and an aryloxy group having 6-10 carbon atoms.

(3) The compounds having the formula (I) or salts thereof, in which $R^1$ represents a phenyl or pyridyl group which may have a substituent selected from the group and atom consisting of an alkyl group having 1-8 carbon atoms, a halogen-substituted alkyl group having 1-8 carbon atoms, an alkoxy group having 1-8 carbon atoms, carboxyl, a halogen atom, hydroxyl, nitro, cyano, or amino.

(4) The compounds having the formula (I) or the compounds of any one of the above-mentioned (1) to (3), or salts thereof, in which $R^2$ represents cyano or nitro.

(5) The compounds having the formula (I) or the compounds of any one of the above-mentioned (1) to (3), or salts thereof, in which $R^2$ represents cyano.

(6) The compounds having the formula (I) or the compounds of any one of the above-mentioned (1) to (5), or salts thereof, in which X is oxygen, NH or sulfur.

(7) The compounds having the formula (I) or the compounds of any one of the above-mentioned (1) to (5), or salts thereof, in which X is oxygen or sulfur.

(8) The compounds having the formula (I) or the compounds of any one of the above-mentioned (1) to (7), or salts thereof, in which Y is sulfur or NH.

(9) The compounds having the formula (I) or the compounds of any one of the above-mentioned (1) to (7), or salts thereof, in which Y is sulfur.

(10) The compounds having the formula (I) or salts thereof, in which $R^1$ represents a phenyl or pyridyl group which may have a substituent selected from the group and atom consisting of an alkyl group having 1-8 carbon atoms, a halogen-substituted alkyl group having 1-8 carbon atoms, an alkoxy group having 1-8 carbon atoms, carboxyl, a halogen atom, hydroxyl, nitro, cyano, or amino; $R^2$ represents cyano or nitro; X is oxygen or sulfur; and Y is sulfur or NH.

(11) The compounds having the formula (I) or salts thereof, in which $R^1$ represents a phenyl or pyridyl group which may have a substituent selected from the group and atom consisting of an alkyl group having 1-8 carbon atoms, a halogen-substituted alkyl group having 1-8 carbon atoms, an alkoxy group having 1-8 carbon atoms, carboxyl, a halogen atom, hydroxyl, nitro, cyano, or amino; $R^2$ represents cyano or nitro; X is oxygen or sulfur; and Y is sulfur.

(12) The compounds having the formula (I) or salts thereof, in which $R^1$ represents a phenyl or pyridyl group which may have a substituent selected from the group and atom consisting of an alkyl group having 1-8 carbon atoms, a halogen-substituted alkyl group having 1-8 carbon atoms, an alkoxy group having 1-8 carbon atoms, carboxyl, a halogen atom, hydroxyl, nitro, cyano, or amino; $R^2$ represents cyano or nitro; X is oxygen; and Y is sulfur.

Processes for preparing compounds having the formula (I) are illustrated below.

[Synthesis process 1 -- in the case of Y = S]

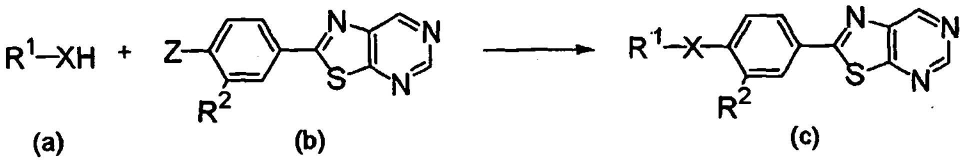

(a) (b) (c)

[in the formulas, Z is a halogen atom such as chlorine, and each of $R^1$, $R^2$ and X has the same meaning as above.]

The compound of the invention represented by the formula (c) can be obtained by reacting a compound of the formula (a) and a 2-phenylthiazolo[5,4-d]pyrimidine derivative of the formula (b).

In the case of X=O, the reaction can be carried out in such a solvent as DMSO at a temperature of from room temperature to 60°C in the presence of a base such as sodium hydride.

In the case of X=S, the reaction can be carried out by refluxing under heating in such a solvent as ethanol in the presence of a base such as potassium carbonate.

In the case of X=NH, the reaction can be carried out by refluxing under heating in the presence of such a base as copper oxide or potassium carbonate.

The starting compound, i.e., a 2-phenylthiazolo[5,4-d]pyrimidine derivative represented by the formula (b), can be obtained, for instance, by the following process.

Synthesis process for the starting compound

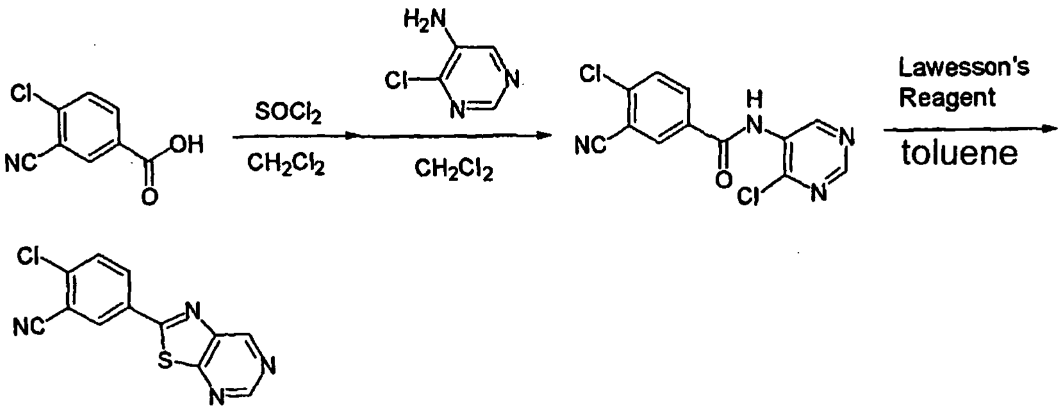


(Synthesis process 2)

In the case of Y = O

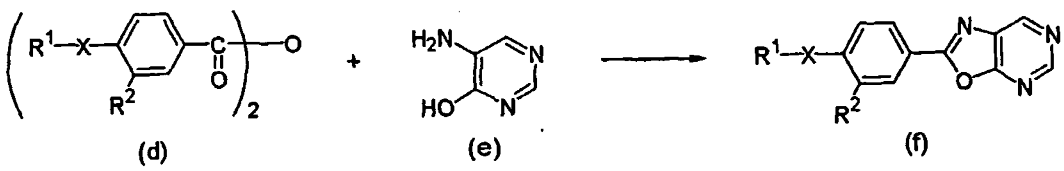

(d) (e) (f)

[in the formulas, each of $R^1$, $R^2$ and X has the same meaning as above.]

The compound of the invention represented by the formula (f) can be obtained by heating the benzoic anhydride of the formula (d) and the 5-amino-4-hydroxypyrimidine derivative of the formula (e).

The benzoic anhydride of the starting compound represented by the formula (d) can be obtained by the following process.

Synthesis process for the starting compound (X = O)

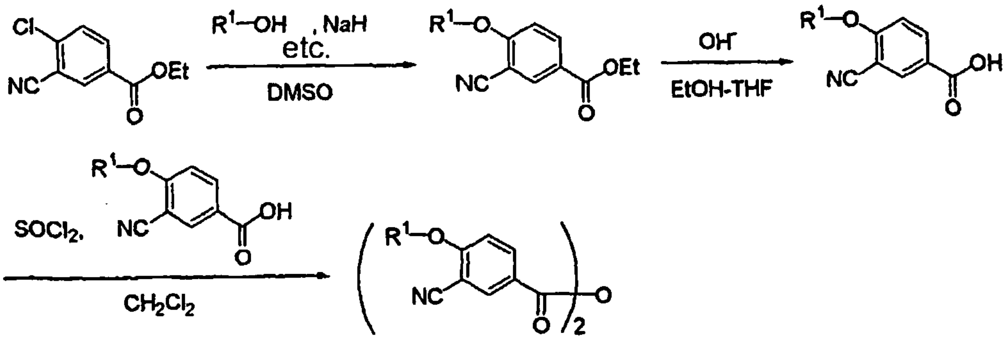


[in the formulas, $R^1$ has the same meaning as above.]

(Synthesis process 3)

In the case of Y = NH

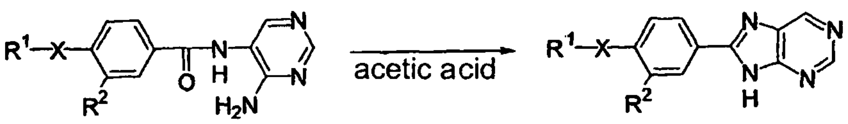


(g) (h)

[in the formulas, each of $R^1$, $R^2$ and X has the same meaning as above.]

The compound of the invention represented by the formula (h) can be obtained by heating the pyrimidine derivative of the formula (g) in the presence of acetic acid.

The benzoic anhydride of the starting compound represented by the formula (g) can be obtained by the following process.

Synthesis process for the starting compound (X = O)

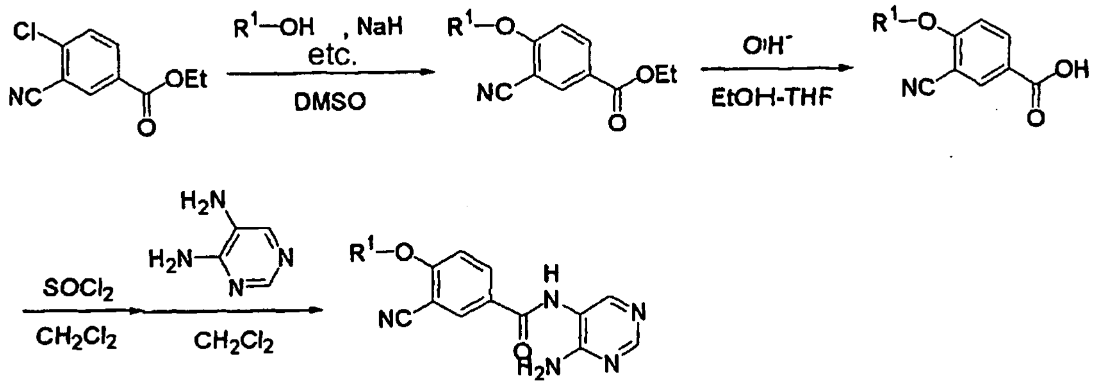


[in the formulas, $R^1$ has the same meaning as above.]

The compounds of the invention can be prepared by the above-mentioned synthesis processes 1 to 3, the working examples described hereinafter, the processes of the Patent Publication 5, etc., and the known processes.

Examples of the compounds of the invention prepared by these processes are set forth in Table 1 to 10:

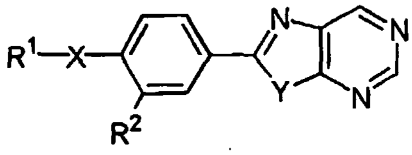

( I )

Each of $R^1$, $R^2$ X and Y is described in Tables 1 to 10.

Table 1

| $R^1$ | $R^2$ | X | Y |
|---|---|---|---|
| phenyl | CN | O | S |
| phenyl | CN | S | S |
| phenyl | CN | O | O |
| phenyl | CN | O | NH |
| phenyl | $NO_2$ | O | S |
| phenyl | $CO_2H$ | O | S |
| phenyl | $CONH_2$ | O | S |
| phenyl | $CO_2Et$ | O | S |
| phenyl | CHO | O | S |
| 3-fluorophenyl | CN | O | S |
| 3-fluorophenyl | CN | S | S |
| 3-fluorophenyl | CN | O | O |

Table 2

| $R^1$ | $R^2$ | X | Y |
|---|---|---|---|
| 3-fluorophenyl | CN | O | NH |

(continued)

| $R^1$ | $R^2$ | X | Y |
|---|---|---|---|
| 4-trifluoromethylphenyl | CN | O | S |
| 4-trifluoromethylphenyl | CN | S | S |
| 4-trifluoromethylphenyl | CN | O | O |
| 4-trifluoromethylphenyl | CN | O | NH |
| 2-furyl | CN | O | S |
| 2-furyl | CN | S | S |
| 2-furyl | CN | O | O |
| 2-furyl | CN | O | NH |
| 2-pyridyl | CN | O | S |
| 2-pyridyl | CN | S | S |
| 2-pyridyl | CN | O | O |

Table 3

| $R^1$ | $R^2$ | X | Y |
|---|---|---|---|
| 2-pyridyl | CN | O | NH |
| 4-fluorophenyl | CN | O | S |
| 4-fluorophenyl | CN | S | S |
| 4-fluorophenyl | CN | O | O |
| 4-fluorophenyl | CN | O | NH |
| 4-fluorophenyl | $NO_2$ | O | S |
| 4-fluorophenyl | $CO_2H$ | O | S |
| 4-fluorophenyl | $CONH_2$ | O | S |
| 4-fluorophenyl | $CO_2Et$ | O | S |
| 4-fluorophenyl | CHO | O | S |
| 2-fluorophenyl | CN | O | S |
| 2-fluorophenyl | CN | S | S |

Table 4

| $R^1$ | $R^2$ | X | Y |
|---|---|---|---|
| 2-fluorophenyl | CN | O | O |
| 2-fluorophenyl | CN | O | NH |
| 4-methoxycarbonylphenyl | CN | O | S |
| 4-methoxycarbonylphenyl | CN | S | S |
| 4-methoxycarbonylphenyl | CN | O | O |
| 4-methoxycarbonylphenyl | CN | O | NH |
| 2-thienyl | CN | O | S |
| 2-thienyl | CN | S | S |
| 2-thienyl | CN | O | O |
| 2-thienyl | CN | O | NH |
| 4-pyridyl | CN | O | S |
| 4-pyridyl | CN | S | S |

Table 5

| $R^1$ | $R^2$ | X | Y |
|---|---|---|---|
| 4-pyridyl | CN | O | O |

(continued)

| $R^1$ | $R^2$ | X | Y |
|---|---|---|---|
| 4-pyridyl | CN | O | NH |
| 4-chlorophenyl | CN | O | S |
| 4-chlorophenyl | CN | S | S |
| 4-chlorophenyl | CN | O | O |
| 4-chlorophenyl | CN | O | NH |
| 4-chlorophenyl | $NO_2$ | O | S |
| 4-chlorophenyl | $CO_2H$ | O | S |
| 4-chlorophenyl | $CONH_2$ | O | S |
| 4-chlorophenyl | $CO_2Et$ | O | S |
| 4-chlorophenyl | CHO | O | S |
| 3-chlorophenyl | CN | O | S |

Table 6

| $R^1$ | $R^2$ | X | Y |
|---|---|---|---|
| 3-chlorophenyl | CN | s | S |
| 3-chlorophenyl | CN | O | O |
| 3-chlorophenyl | CN | O | NH |
| 4-carboxyphenyl | CN | O | S |
| 4-carboxyphenyl | CN | s | S |
| 4-carboxyphenyl | CN | O | O |
| 4-carboxyphenyl | CN | O | NH |
| 2-pyrrolyl | CN | O | S |
| 2-pyrrolyl | CN | s | S |
| 2-pyrrolyl | CN | O | O |
| 2-pyrrolyl | CN | O | NH |
| 2-hydroxyphenyl | CN | O | S |

Table 7

| $R^1$ | $R^2$ | X | Y |
|---|---|---|---|
| 2-hydroxyphenyl | CN | S | S |
| 2-hydroxyphenyl | CN | O | O |
| 2-hydroxyphenyl | CN | O | NH |
| 2-hydroxyphenyl | $NO_2$ | O | S |
| 2-hydroxyphenyl | $CO_2H$ | O | S |
| 2-hydroxyphenyl | $CONH_2$ | O | S |
| 2-hydroxyphenyl | $CO_2Et$ | O | S |
| 2-hydroxyphenyl | CHO | O | S |
| 2-chlorophenyl | CN | O | S |
| 2-chlorophenyl | CN | S | S |
| 2-chlorophenyl | CN | O | O |
| 2-chlorophenyl | CN | O | NH |

Table 8

| $R^1$ | $R^2$ | X | Y |
|---|---|---|---|
| 4-hydroxyphenyl | CN | O | S |
| 4-hydroxyphenyl | CN | s | S |

(continued)

| $R^1$ | $R^2$ | X | Y |
|---|---|---|---|
| 4-hydroxyphenyl | CN | O | O |
| 4-hydroxyphenyl | CN | O | NH |
| 4-imidazolyl | CN | O | S |
| 4-imidazolyl | CN | S | S |
| 4-imidazolyl | CN | O | O |
| 4-imidazolyl | CN | O | NH |
| 3-pyridyl | CN | O | S |
| 3-pyridyl | CN | S | S |
| 3-pyridyl | CN | O | O |
| 3-pyridyl | CN | O | NH |

Table 9

| $R^1$ | $R^2$ | X | Y |
|---|---|---|---|
| 3-pyridyl | $NO_2$ | O | S |
| 3-pyridyl | $CO_2H$ | O | S |
| 3-pyridyl | $CONH_2$ | O | S |
| 3-pyridyl | $CO_2Et$ | O | S |
| 3-pyridyl | CHO | O | S |
| 4-methoxyphenyl | CN | O | S |
| 4-methoxyphenyl | CN | S | S |
| 4-methoxyphenyl | CN | O | O |
| 4-methoxyphenyl | CN | O | NH |
| 3-hydroxyphenyl | CN | O | S |
| 3-hydroxyphenyl | CN | S | S |
| 3-hydroxyphenyl | CN | O | O |

Table 10

| $R^1$ | $R^2$ | X | Y |
|---|---|---|---|
| 3-hydroxyphenyl | CN | O | NH |
| 2-thiazolyl | CN | O | S |
| 2-thiazolyl | CN | S | S |
| 2-thiazolyl | CN | O | O |
| 2-thiazolyl | CN | O | NH |

The pharmacological actions of the compounds of the invention are described below.

The xanthine oxidase inhibiting action (*in vitro* test) of the compound of the invention was confirmed by measuring inhibition of oxidation of xanthine by xanthine oxidase, as described in Example 26. As is clear from Tables 11 and 12, the compounds of the invention show excellent xanthine oxidase inhibiting action.

The xanthine oxidase inhibiting action was further confirmed in vivo tests by measuring the uric acid concentration in a plasma obtained from mouse into which the compound of the invention had been orally administered. See Example 27 and Tables 11 and 12.

Accordingly, it is expected that the compounds of the invention having the formula (I) are employable for preventing or treating hyperuricemia and gout.

The compounds of the invention can be administered into human beings by appropriate administration methods such as oral administration and parenteral administration.

The compounds of the invention can be prepared in the form of known pharmaceutical preparations such as pellets, granules, powders, capsules, suspensions, injections, and suppositories. For the preparations, a conventionally employed excipients, disintegrators, binder, lubricants, dyes, diluents, or the like are employed. The excipient may be

lactose, D-mannitol, crystalline cellulose, or glucose. The disintegrator may be starch or carboxymethylcellulose calcium (CMC-Ca). The lubricant may be magnesium stearate or talc. The binder may be hydroxypropylcellulose (HPC), gelatin, or polyvinylpyrrolidone (PVP).

The dosage of the compound of the invention for adult generally is approximately 0.1 to 100 mg/day when it is administered in the form of an injection, and approximately 1 to 2,000 mg/day when it is orally administered. The dosage can be adjusted depending on age and clinical conditions.

The present invention is further described below by the following non-limiting examples and reference examples.

[Example 1]

(1) 4-Chloro-N-(4-chloro-5-pyrimidinyl)-3-cyanobenzamide

4-Chloro-3-cyanobenzoic acid (7.01 g, 38.6 mmol) was suspended in benzene (70 mL), and thionyl chloride (3.6 mL, 49.6 mmol) was added to the resulting suspension. The suspension was then heated under reflux for 4 hours. The reaction mixture was concentrated under reduced pressure. To the resulting acid chloride were added 5-amino-4-chloropyrimidine (5.00 g, 38.6 mmol), dichloromethane (70 mL) and pyridine (3.6 mL, 44.5 mmol). The resulting mixture was stirred for 7 hours at room temperature. To the reaction mixture were added chloroform (50 mL) and water (50 mL), and the precipitated crystalline product was collected by filtration, washed with chloroform (20 mL) and water (20 mL), and dried in air, to obtain 7.35 g (yield 65%) of the titled compound as a white crystalline product. The mother liquor and a mixture of washings were processed to obtain 0.62 g (yield 8%) of the titled compound as a pale brown crystalline product (second crystalline product). Total yield 73%.
m.p.: 189-190°C
$^1$H-NMR ($CDCl_3$, 400 MHz) δ: 7.74 (1H, d, J=8Hz), 8.07 (1H, dd, J=2Hz, 8Hz), 8.13 (1H, s), 8.23 (1H, d, J=2Hz), 8.83 (1H, s), 9.79 (1H, s).

(2) 2-(4-Chloro-3-cyanophenyl)thiazolo[5,4-d]pyrimidine

A suspension of the above-mentioned 4-chloro-N-(4-chloro-5-pyrimidinyl)-3-cyanobenzamide (7.98 g, 27.2 mmol) and Lawesson's reagent (8.25 g, 20.4 mmol) in toluene (150 mL) was heated under reflux for 8 hours, and allowed to stand to cool to room temperature. The crystalline precipitate was collected by filtration, washed with chloroform (75 mL x 2), and dried in air, to obtain 7.25 g (yield 98%) of the titled compound as a pale yellow crystalline product.
m.p.: 278-280°C (decomp.)
$^1$H-NMR (DMSO-$d_6$, 400 MHz) δ: 7.99 (1H, d, J=9Hz), 8.47 (1H, dd, J=2Hz, 9Hz), 8.70 (1H, d, J=2Hz), 9.20 (1H, s), 9.54 (1H, s).

(3) 2-[4-(4-fluorophenoxy)-3-cyanophenyl]thiazolo[5,4-d]pyrimidine

To a suspension of 55% sodium hydride (150 mg, 3.44 mmol) in dry DMSO (7 mL) was added 4-fluorophenol (383 mg, 3.42 mmol). The mixture was stirred for 30 minutes under heating to 50°C. To the reaction mixture was added the above-mentioned 2-(4-chloro-3-cyanophenyl)thiazolo-[5,4-d]pyrimidine (776 mg, 2.85 mmol), and the resulting mixture was stirred at 50°C for 4 hours. Thus obtained reaction mixture was allowed to stand to cool to room temperature, and water (35 mL) was added to the mixture. The crystalline precipitate was collected by filtration, washed with water (20 mL) and dried in air. The resulting crystalline product was purified by silica gel column chromatography (chloroform) and washed with ether (15 mL), to obtain 701 mg (yield 71%) of the titled compound as a pale yellow crystalline product.
m.p.: 175-177°C
$^1$H-NMR ($CDCl_3$, 400 MHz) δ: 6.94 (1H, d, J=9Hz), 7.1-7.2 (4H, m), 8.18 (1H, dd, J=2Hz, 9Hz), 8.44 (1H, d, J=2Hz), 9.13 (1H, s), 9.35 (1H, s).
IR (KBr) $cm^{-1}$: 2233, 1606, 1564, 1419, 1300, 1119, 1011, 916, 893, 847, 829, 777, 760, 758, 723, 702, 700, 650, 648, 597, 526, 496, 490.
FAB-MS (m/e): 349 (M+1).

[Example 2] 2-[3-Cyano-4-phenoxyphenyl)thiazolo[5,4-d]-pyrimidine

To a suspension of 55% sodium hydride (23 mg, 0.53 mmol) in dry DMSO (1 mL) was added phenol (45 mg, 0.48 mmol). The mixture was stirred for 30 minutes at room temperature. To the reaction mixture was added the above-mentioned 2-(4-chloro-3-cyanophenyl)thiazolo[5,4-d]pyrimidine (120 mg, 0.44 mmol), and the resulting mixture was stirred at 60°C for 4 hours. Thus obtained reaction mixture was allowed to stand to cool to room temperature, and water (5 mL) was added to the mixture. The crystalline precipitate was collected by filtration, washed successively with water

(5 mL), ethanol (1 mL) and ether (2 mL), and dried under reduced pressure in air, to obtain 100 mg (yield 69%) of the titled compound as a pale brown crystalline product.
m.p.: 154-156°C
$^1$H-NMR (DMSO-$d_6$, 400 MHz) δ: 7.08 (1H, d, J=9Hz), 7.2-7.4 (3H, m), 7.5-7.6 (2H, m), 8.42 (1H, dd, J=2Hz, 9Hz), 8.69 (1H, d, J=2Hz), 9.18 (1H, s), 9.52 (1H, s).
IR (KBr) $cm^{-1}$: 3037, 2227, 1605, 1587, 1560, 1525, 1504, 1470, 1369, 1365, 1257, 1238, 1190, 1171.
FAB-MS (m/e): 331 (M+1).

[Example 3] 2-[3-Cyano-4-(3-pyridyloxy)phenyl]thiazolo-[5,4-d]pyrimidine

To a suspension of 60% sodium hydride (24 mg, 0.60 mmol) in dry DMSO (1 mL) was added 3-hydroxypyrimidine (57 mg, 0.60 mmol). The mixture was stirred for 1.5 hours at room temperature. To the reaction mixture was added the above-mentioned 2-(4-chloro-3-cyanophenyl)-thiazolo[5,4-d]pyrimidine (82 mg, 0.30 mmol), and the resulting mixture was stirred for 87 hours at room temperature. To thus obtained reaction mixture was added water (4 mL) under ice-cooling, and the mixture was stirred for 15 minutes. The crystalline precipitate was collected by filtration and washed with water to obtain a brown crystalline product. The crystalline product was purified by silica gel column chromatography (methanol/chloroform), to obtain 59 mg (yield 60%) of the titled compound as a pale yellow crystalline product.
m.p.: 210-212°C
$^1$H-NMR ($CDCl_3$, 400 MHz) δ: 6.99 (1H, d, J=9Hz), 7.45 (1H, dd, J=5Hz, 8Hz), 7.53 (1H, ddd, J=1Hz, 3Hz, 8Hz), 8.22 (1H, dd, J=2Hz, 9Hz), 8.47 (1H, d, J=2Hz), 8.56 (1H, d, J=3Hz), 8.60 (1H, dd, J=1Hz, 5Hz), 9.14 (1H, s), 9.36 (1H, s).
IR (KBr) $cm^{-1}$: 2229, 1610, 1568, 1502, 1475, 1427, 1396, 1373, 1265, 1259, 1203, 1107.
FAB-MS (m/e) : 332 (M+1).

[Example 4]

(1) 3-Cyano-4-(4-fluorophenoxy)benzoic acid

To a solution of 4-chloro-3-cyanobenzoic acid (545 mg, 3.00 mmol) and 4-fluorophenol (504 mg, 4.50 mmol) in dry DMSO (6 mL) was added 60% sodium hydride (300 mg, 7.50 mmol). The mixture was stirred for 15 minutes at room temperature, and subsequently for 9 hours at 70°C. The reaction mixture was cooled to room temperature, and water was added. The aqueous mixture was adjusted to approx. pH 6 by addition of 6N hydrochloric acid and subjected to exaction with ethyl acetate (2 portions). The extracts were combined, washed with water and saturated brine, dried over anhydrous sodium sulfate, and placed under reduced pressure to distill the solvent off. The residue was heated under reflux for 5 minutes after addition of ethyl acetate (6 mL), and stirred for one hour at room temperature. The crystalline precipitate was collected by filtration, washed with ethyl acetate, and dried in air, to obtain 170 mg (yield 22%) of the titled compound as a pale brown crystalline compound.
$^1$H-NMR ($CDCl_3$/$CD_3OD$=8/1, 400 MHz) δ: 6.82 (1H, d, J=9Hz), 7.1-7.3 (4H, m), 8.14 (1H, dd, J=2Hz, 9Hz), 8.37 (1H, d, J=2Hz).

(2) N-(4-Amino-5-pyrimidinyl)-3-cyano-4-(4-fluorophenoxy)benzamide

Thionyl chloride (2 mL) was added to the above-mentioned 3-cyano-4-(4-fluorophenoxy)benzoic acid (143 mg, 0.56 mmol), and the mixture was heated overnight under reflux. The reaction mixture was cooled to room temperature and concentrated under reduced pressure. The residue was subjected three times to a procedure of adding benzene (5 mL) and concentrating under reduced pressure. Thus obtained acid chloride was dissolved in pyridine (3 mL). The pyridine solution was dropwise added under ice-cooling to a suspension of 4,5-diaminopyridine (612 mg, 5.56 mmol) in pyridine (6 mL) for 15 minutes. The mixture was stirred at 5°C for 6 hours and at room temperature for 15 hours. The reaction mixture was subjected to extraction with ethyl acetate (2 portions) after addition of water (90 mL). The extracts were combined, washed successively with water, saturated aqueous sodium hydrogen carbonate and saturated brine, dried over anhydrous sodium sulfate, and placed under reduced pressure to distill the solvent off. The residue was stirred for 15 minutes at room temperature after addition of ether (4 mL). The crystalline precipitate was collected by filtration, washed with ether, and dried in air, to obtain 80 mg (yield 41%) of the titled compound as a pale brown crystalline product.
$^1$H-NMR (DMSO-$d_6$, 400 MHz) δ: 6.90 (2H, br s), 6.99 (1H, d, J=9Hz), 7.3-7.5 (4H, m), 8.14 (1H, s), 8.18 (1H, dd, J=2Hz, 9Hz), 8.28 (1H, s), 8.54 (1H, d, J=2Hz), 9.80 (1H, s).
FAB-MS (m/e): 350 (M+1)

(3) 8-[3-Cyano-4-(4-fluorophenoxy)phenyl]-9H-purine

Acetic acid (2 mL) was added to the above-mentioned N-(4-amino-5-pyrimidinyl)-3-cyano-4-(4-fluorophenoxy)-benzamide (76 mg, 0.22 mmol), and the mixture was heated under reflux for 24 hours. The reaction mixture was cooled to room temperature and concentrated under reduced pressure to dryness. The residue was heated under reflux for 5 minutes after addition of ethyl acetate (2 mL), and subsequently stirred for 15 minutes at room temperature. The crystalline precipitate was collected by filtration, washed with ethyl acetate, and dried in air, to obtain 65 mg (yield 90%) of the titled compound as a pale red crystalline product.
m.p.: >250°C
$^1$H-NMR (DMSO-$d_6$, 400 MHz) δ: 7.10 (1H, d, J=9Hz), 7.3-7.4 (4H, m), 8.49 (1H, dd, J=2Hz, 9Hz), 8.68 (1H, d, J=2Hz), 8.91 (1H, s), 9.12 (1H, s).
IR (KBr) $cm^{-1}$: 2867, 2231, 1626, 1589, 1506, 1485, 1435, 1387, 1344, 1281, 1238, 1219, 1188, 1124, 1118, 1020, 966, 924, 864, 843, 816, 793, 785, 731, 712, 613, 584, 553, 532, 499.
FAB-MS (m/e): 332 (M+1)

[Example 5] 2-[3-Cyano-4-(2-fluorophenoxy)phenyl]-thiazolo[5,4-d]pyrimidine

To a suspension of the aforementioned 2-(4-chloro-3-cyanophenyl)thiazolo[5,4-d]pyrimidine (120 mg, 0.44 mmol) in dry DMSO (2 mL) were successively added 2-fluorophenol (123 mg, 1.10 mmol) and 55% sodium hydride (48 mg, 1.10 mmol). The mixture was stirred under heating to 50°C for 5 hours and cooled to room temperature. To the reaction mixture was added water (8 mL). The crystalline precipitate was collected by filtration, washed with water and dried in air. The resulting crystalline product was purified by silica gel column chromatography (ethyl acetate/n-hexane), to obtain 122 mg (yield 80%) of the titled compound as a pale yellow crystalline product.
m.p.: 195-196°C
$^1$H-NMR ($CDCl_3$, 400 MHz) δ: 6.90 (1H, d, J=9Hz), 7.2-7.4 (4H, m), 8.18 (1H, dd, J=2Hz, 9Hz), 8.44 (1H, d, J=2Hz), 9.13 (1H, s), 9.35 (1H, s).
IR (KBr) $cm^{-1}$: 2235, 1608, 1571, 1527, 1500, 1477, 1454, 1400, 1373, 1279, 1240, 1207, 1184, 1151, 1112, 1028, 995, 950, 902, 875, 829, 793, 777, 756, 725, 708, 685, 652, 648, 631, 600, 494.
FAB-MS (m/e): 349 (M+1)

[Example 6] 2-[3-Cyano-4-(3-fluorophenoxy)phenyl]-thiazolo[5,4-d]pyrimidine

To a suspension of the aforementioned 2-(4-chloro-3-cyanophenyl)thiazolo[5,4-d]pyrimidine (120 mg, 0.44 mmol) in dry DMSO (2 mL) were successively added 3-fluorophenol (123 mg, 1.10 mmol) and 55% sodium hydride (48 mg, 1.10 mmol). The mixture was stirred under heating to 50°C for 4 hours and cooled to room temperature. To the reaction mixture was added water (8 mL). The crystalline precipitate was collected by filtration, washed with water and dried in air. The resulting crystalline product was purified by silica gel column chromatography (ethyl acetate/n-hexane), to obtain 119 mg (yield 78%) of the titled compound as a pale yellow crystalline product.
m.p.: 189-190°C
$^1$H-NMR ($CDCl_3$, 400 MHz) δ: 6.8-7.1 (4H, m), 7.4-7.5 (1H, m), 8.21 (1H, dd, J=2Hz, 9Hz), 8.45 (1H, d, J=2Hz), 9.14 (1H, s), 9.36 (1H, s).
IR (KBr) $cm^{-1}$: 2231, 1601, 1572, 1527, 1502, 1473, 1468, 1398, 1375, 1265, 1207, 1159, 1128, 1070, 995, 958, 910, 862, 840, 835, 787, 759, 744, 721, 692, 650.
FAB-MS (m/e): 349 (M+1)

[Example 7] 2-[3-Cyano-4-(4-fluorophenylthio)phenyl]-thiazolo[5,4-d]pyrimidine

To a suspension of 55% sodium hydride (21 mg, 0.48 mmol) in dry DMSO (1 mL) was added 4-fluorothiophenol (0.05 mL, 0.47 mmol). The mixture was stirred for 10 minutes at room temperature. To the reaction mixture was added the aforementioned 2-(4-chloro-3-nitrophenyl)-4-hydroxythiazolo[5,4-d]pyrimidine (120 mg, 0.44 mmol), and the resulting mixture was stirred for 2 hours at room temperature. To thus obtained reaction mixture was added water (5 mL). The crystalline precipitate was collected by filtration; washed successively with water (5 mL x 2), ethanol (1 mL) and ether (5 mL), and dried under reduced pressure at room temperature for one hour, to obtain 126 mg (yield 79%) of the titled compound as a pale yellow crystalline product.
m.p.: 190-195°C
$^1$H-NMR ($CDCl_3$, 400 MHz) δ: 7.02 (1H, d, J=9Hz), 7.1-7.3 (2H, m), 7.5-7.7 (2H, m), 8.05 (1H, dd, J=2Hz, 9Hz), 8.38 (1H, d, J=2Hz), 9.13 (1H, s), 9.35 (1H, s).
IR (KBr) $cm^{-1}$: 2224, 1587, 1591, 1568, 1520, 1491, 1460, 1387, 1383, 1373, 1286, 1232, 1057, 827.

FAB-MS (m/e): 365 (M+1)

[Example 8]

(1) 2-[3-Cyano-4-(2-methoxymethoxy)phenyl]thiazolo[5,4-d]pyrimidine

To a suspension of 55% sodium hydride (48 mg, 1.10 mmol) in dry DMSO (1.5 mL) was added 2-methoxymethoxyphenol (208 mg, purity 81.6%, 1.10 mmol). The mixture was stirred at 50°C for 30 minutes. To the mixture was added the aforementioned 2-(4-chloro-3-cyanophenyl)-thiazolo[5,4-d]pyrimidine (150 mg, 0.55 mmol). The resulting mixture was stirred at 50°C for 17 hours and cooled to room temperature. To thus obtained reaction mixture was added water (7 mL). The crystalline precipitate was collected by filtration, washed with water (5 mL x 2), dried in air, washed with ether (10 mL), and dried under reduced pressure at room temperature, to obtain 146 mg (yield 68%) of the titled compound as a pale brown crystalline product.
m.p.: 149-151°C
$^1$H-NMR ($CDCl_3$, 400 MHz) δ: 3.39 (3H, s), 5.13 (2H, s), 7.2-7.3 (3H, m), 8.13 (1H, dd, J=2Hz, 9Hz), 8.42 (1H, d, J=2Hz), 9.12 (1H, s), 9.33 (1H, s).

(2) 2-[3-Cyano-4-(2-hydroxyphenoxy)phenyl]thiazolo[5,4-d]pyrimidine

The above-mentioned 2-[3-cyano-4-(2-methoxymethoxy)-phenyl]thiazolo[5,4-d]pyrimidine (144 mg, 0.37 mmol) was dissolved in THF (6 mL). After addition of 3 mol/L aqueous hydrochloric acid (0.6, 1.80 mmol), the solution was heated under reflux for 4 hours. The solution was then placed under reduced pressure to distill the solvent off. The residue was purified by silica gel column chromatography (ethyl acetate/n-hexane, methanol/chloroform), to obtain 42 mg (yield 33%) of the titled compound as a pale pink crystalline product.
m.p.: 255-256°C
$^1$H-NMR ($CDCl_3$, 400 MHz) δ: 6.87 (1H, d, J=9Hz), 6.94 (1H, dt, J=1Hz, 7Hz), 7.07 (1H, dd, J=1Hz, 8Hz), 7.1-7.3 (2H, m), 8.37 (1H, dd, J=2H, 9Hz), 8.63 (1H, d, J=2Hz), 9.50 (1H, s), 9.97 (1H, s).
IR (KBr) $cm^{-1}$: 3034, 2235, 1610, 1578, 1527, 1495, 1475, 1400, 1377, 1281, 1213, 1111.
FAB-MS (m/e): 347 (M+1)

[Example 9] 2-[4-(4-Chlorophenyloxy)-3-cyanophenyl]-thiazolo[5,4-d]pyrimidine

To a suspension of 60% sodium hydride (36 mg, 0.90 mmol) in dry dimethyl sulfoxide (2 mL) was added 4-chlorophenol (116 mg, 0.90 mmol). The mixture was stirred for one hour at room temperature. To the mixture was added the aforementioned 2-(4-chloro-3-cyanophenyl)-thiazolo[5,4-d]pyrimidine (82 mg, 0.30 mmol), and the resulting mixture was stirred for 48 hours at room temperature. To thus obtained reaction mixture was added water (8 mL) under ice-cooling, and the mixture was stirred for 10 minutes. The crystalline precipitate was collected by filtration and washed with water to obtain a brown crystalline product. The crystalline product was purified by silica gel column chromatography (chloroform), to obtain 90 mg (yield 83%) of the titled compound as a white crystalline product.
m.p.: 179-180°C
$^1$H-NMR ($CDCl_3$, 400 MHz) δ: 6.97 (1H, d, J=9Hz), 7.12 (2H, d, J=9Hz), 7.45 (2H, d, J=9Hz), 8.19 (1H, dd, J=2Hz, 9Hz), 8.44 (1H, d, J=2Hz), 9.14 (1H, s), 9.35 (1H, s).
IR (KBr) $cm^{-1}$: 2229, 1608, 1568, 1566, 1504, 1485, 1477, 1398, 1379, 1271, 1198, 1086.
FAB-MS (m/e): 365 (M+1)

[Example 10] 2-[3-Cyano-4-(2-fluorophenylthio)phenyl]-thiazolo[5,4-d]pyrimidine

To a suspension of 55% sodium hydride (23 mg, 0.53 mmol) in dry DMSO (1 mL) was added 2-fluorophenol (68 mg, 0.53 mmol). The mixture was stirred at 50°C for 30 minutes. To the reaction mixture was added the aforementioned 2-(4-chloro-3-cyanophenyl)thiazolo[5,4-d]pyrimidine (120 mg, 0.44 mmol). The resulting mixture was stirred at 50°C for 5 hours and cooled to room temperature. To thus obtained reaction mixture was added water (5 mL). The crystalline precipitate was collected by filtration and washed successively with water (5 mL), ethanol (1 mL) and ether (3 mL), to obtain 123 mg (yield 77%) of the titled compound as a pale orange crystalline product.
m.p.: 178-180°C
$^1$H-NMR (DMSO-$d_6$, 400 MHz) δ: 7.21 (1H, d, J=8Hz), 7.3-7.6 (2H, m), 7.6-7.8 (2H, m), 8.34 (1H, dd, J=2Hz, 8Hz), 8.65 (1H, d, J=2Hz), 9.19 (1H, s), 9.53 (1H, s).
IR (KBr) $cm^{-1}$: 2225, 1589, 1571, 1525, 1473, 1450, 1383, 1261, 1221, 1055, 827, 795, 764, 723, 681, 651, 598, 476, 474.
FAB-MS (m/e): 365 (M+1)

[Example 11] 2-(3-Cyano-4-phenylthiophenyl)thiazolo[5,4-d]pyrimidine

To a suspension of 55% sodium hydride (23 mg, 0.53 mmol) in dry DMSO (1 mL) was added thiophenol (58 mg, 0.53 mmol). The mixture was stirred at 50°C for 30 minutes. To the reaction mixture was added the aforementioned 2-(4-chloro-3-cyanophenyl)thiazolo[5,4-d]pyrimidine (120 mg, 0.44 mmol). The resulting mixture was stirred at 50°C for 5 hours and cooled to room temperature. To thus obtained reaction mixture was added water (4 mL). The crystalline precipitate was collected by filtration and washed successively with water (5 mL), ethanol (1 mL) and ether (3 mL), to obtain 120 mg (yield 79%) of the titled compound as a pale yellow crystalline product.
m.p.: 173-176°C
$^1$H-NMR (DMSO-$d_6$, 400 MHz) δ : 7.19 (1H, d, J=9Hz), 7.5-7.7 (5H, m), 8.32 (1H, dd, J=2Hz, 9Hz), 8.62 (1H, d, J=2Hz), 9.19 (1H, s), 9.52 (1H, s).
IR (KBr) $cm^{-1}$: 3051, 2224, 1597, 1568, 1524, 1460, 1385, 1304, 1284, 1252, 1238, 1205, 1055, 1024, 995, 906, 831, 812, 744, 685, 650, 598, 488, 472.
FAB-MS (m/e): 347 (M+1)

[Example 12] 2-(4-Allyloxy-3-cyanophenyl)thiazolo[5,4-d]-pyrimidine

To a suspension of 55% sodium hydride (26 mg, 0.60 mmol) in dry DMSO (1 mL) was added allyl alcohol (0.04 mL, 0.59 mmol). The mixture was stirred at 50°C for 30 minutes. To the reaction mixture was added the aforementioned 2-(4-chloro-3-nitrophenyl)-4-hydroxythiazolo-[5,4-d]pyrimidine (120 mg, 0.44 mmol). The resulting mixture was stirred at 50°C for 27 hours. After addition of water (5 mL), the resulting crystalline precipitate was collected by filtration, washed successively with water (5 mL), ethanol (1 mL) and ether (2 mL), and dried in air, to obtain 70 mg (yield 54%) of the titled compound as a pale yellow crystalline product.
m.p.: 202.6-207.6°C
$^1$H-NMR ($CDCl_3$, 400 MHz) δ: 4.79 (2H, dt, J=1Hz, 5Hz), 5.41 (1H, dd, J=1Hz, 10Hz), 5.53 (1H, dd, J=1Hz, 17Hz), 6.0-6.2 (1H, m), 7.13 (1H, d, J=9Hz), 8.25 (1H, dd, J=2Hz, 9Hz), 8.36 (1H, d, J=2Hz), 9.12 (1H, s), 9.33 (1H, s).
IR (KBr) $cm^{-1}$: 2999, 2227, 1610, 1574, 1527, 1512, 1481, 1458, 1450, 1406, 1373, 1290, 1288, 1261, 1234, 1205, 1122, 989, 926.
FAB-MS (m/e): 295 (M+1)

[Example 13] 2-(3-Cyano-4-morpholin-4-ylphenyl)thiazolo-[5,4-d]pyrimidine

A suspension of the aforementioned 2-(4-chloro-3-cyanophenyl)thiazolo[5,4-d]pyrimidine (120 mg, 0.44 mmol) in morpholine (1 mL) was stirred at 60°C for 17 hours and at 80°C for 5 hours, and cooled to room temperature. To the reaction mixture was added water (4 mL). The crystalline precipitate was collected by filtration, and washed successively with water (5 mL), ethanol (1 mL) and ether (3 mL). The washed crystalline product was purified by silica gel column chromatography (hexane/ethyl acetate=1/2), to obtain 62 mg (yield 41%) of the titled compound as a pink crystalline product.
m.p.: 232-234°C (decomp.)
$^1$H-NMR (DMSO-$d_6$, 400 MHz) δ: 3.40 (4H, bt), 3.80 (4H, bt), 7.34 (1H, d, J=9Hz), 8.32 (1H, dd, J=2Hz, 9Hz), 8.43 (1H, d, J=2Hz), 9.14 (1H, s), 9.46 (1H, s).
IR (KBr) $cm^{-1}$: 2218, 1605, 1566, 1522, 1479, 1448, 1400, 1375, 1302, 1234, 1120, 1113, 1049, 926, 895, 825, 760, 723, 665, 646, 625, 596, 515.
FAB-MS (m/e): 324 (M+1)

[Example 14] 2-[3-Cyano-4-(4-methyl-1-piperazinyl)-phenyl]thiazolo[5,4-d]pyrimidine

A mixture of 2-(4-chloro-3-cyanophenyl)thiazolo[5,4-d]pyrimidine (120 mg, 0.44 mmol) and 1-methylpiperazine (0.49 mL) was stirred under heating at 80°C for 10 hours and cooled to room temperature. The reaction mixture was then purified by silica gel column chromatography (chloroform/methanol=25/1), to obtain 69 mg (yield 47%) of the titled compound as a pale orange crystalline product.
m.p.: 163-165°C (decomp.)
$^1$H-NMR ($CDCl_3$, 400 MHz) δ: 2.39 (3H, s), 2.6-2.7 (4H, m), 3.4-3.5 (4H, m), 7.09 (1H, d, J=9Hz), 8.16 (1H, dd, J=2Hz, 9Hz), 8.30 (1H, d, J=2Hz), 9.09 (1H, s), 9.30 (1H, s).
IR (KBr) $cm^{-1}$: 2214, 1610, 1570, 1525, 1473, 1448, 1404, 1373, 1294, 1250, 1201, 1144, 1110, 1051, 1005, 943, 926, 924, 812, 787, 758, 723, 681, 667, 646, 627.
FAB-MS (m/e): 337 (M+1)

[Example 15] 2-[4-(3-Chlorophenyloxy)-3-cyanophenyl]-thiazolo[5,4-d]pyrimidine

To a suspension of 60% sodium hydride (48 mg, 1.20 mmol) in dry dimethyl sulfoxide (3 mL) was added 3-chlorophenol (154 mg, 1.20 mmol). The mixture was stirred for one hour at room temperature. After addition of 2-(4-chloro-3-cyanophenyl)thiazolo[5,4-d]pyrimidine (82 mg, 0.30 mmol), the mixture was stirred at 50°C for 20 hours. To the mixture was then added water (15 mL) under ice-cooling. The crystalline precipitate was collected by filtration and washed successively with 4 portions of water (3 mL), 3 portions of ethanol (3 mL) and 3 portions of hexane (3 mL), to obtain 80 mg (yield 73%) of the titled compound as a pale brown crystalline product.
m.p.: 171-173°C
$^1$H-NMR (DMSO-$d_6$, 400 MHz) δ: 7.19 (1H, d, J=9Hz), 7.30 (1H, d, J=8Hz), 7.44 (1H, d, J=8Hz), 7.50 (1H, s), 7.57 (1H, dd, J=8Hz, 8Hz), 8.44 (1H, d, J=9Hz), 8.71 (1H, s), 9.19 (1H, s), 9.53 (1H, s).
IR (KBr) $cm^{-1}$: 2233, 1608, 1574, 1527, 1502, 1473, 1398, 1377, 1269, 1196, 1111, 916.
FAB-MS (m/e): 365 (M+1)

[Example 16] 2-[3-Cyano-4-(thiomorpholin-4-yl)phenyl]-thiazolo[5,4-d]pyrimidine

To the aforementioned 2-(4-chloro-3-cyanophenyl)-4-hydroxythiazolo[5,4-d]pyrimidine (120 mg, 0.44 mmol) was added thiomorpholine (1 mL, 9.94 mmol). The mixture was stirred at 60°C for 46 hours. To the mixture were then added water (5 mL) and chloroform (5 mL). The insolubles were removed by filtration. The organic portion was dried over anhydrous sodium sulfate and placed under reduced pressure to distill the solvent off. The residue was purified by silica gel column chromatography (n-hexane/ethyl acetate), to obtain 17. mg (yield 11%) of the titled compound as an orange crystalline product.
m.p.: 170-175°C (decomp.)
$^1$H-NMR ($CDCl_3$, 400 MHz) δ: 2.8-3.0 (4H, m), 3.6-3.7 (4H, m), 7.11 (1H, d, J=9Hz), 8.18 (1H, dd, J=2Hz, 9Hz), 8.32 (1H, d, J=2Hz), 9.10 (1H, s), 9.31 (1H, s).
IR (KBr) $cm^{-1}$: 2850, 2220, 1606, 1566, 1524, 1520, 1477, 1452, 1400, 1373, 1290, 1232, 1192.
FAB-MS (m/e): 339 (M+1)

[Example 17] 2-[4-(2-Chlorophenyloxy)-3-cyanophenyl]-thiazolo[5,4-d]pyrimidine

To a suspension of 60% sodium hydride (48 mg, 1.20 mmol) in dry dimethyl sulfoxide (3 mL) was added 2-chlorophenol (154 mg, 1.20 mmol), and the mixture was stirred for one hour at room temperature. After addition of 2-(4-chloro-3-cyanophenyl)thiazolo[5,4-d]pyrimidine (82 mg, 0.30 mmol), the mixture was stirred at 50°C for 42 hours. To the reaction mixture was added water (12 mL) under ice-cooling. The crystalline precipitate was collected by filtration and washed successively with 4 portions of water (3 mL), 2 portions of ethanol (3 mL) and 2 portions of hexane (3 mL), to obtain 54 mg (yield 50%) of the titled compound as a pale yellow crystalline product.
m.p.: 209-211°C
$^1$H-NMR (DMSO-$d_6$, 400 MHz) δ: 6.96 (1H, d, J=9Hz), 7.4-7.6 (3H, m), 7.74 (1H, d, J=9Hz), 8.41 (1H, dd, J=2Hz, 9Hz), 8.72 (1H, d, J=2Hz), 9.19 (1H, s), 9.52 (1H, s).
IR (KBr) $cm^{-1}$: 2235, 1608, 1568, 1527, 1502, 1477, 1398, 1379, 1238, 1207, 1109, 1061.
FAB-MS (m/e): 365 (M+1)

[Example 18]

(1) N-(4-Hydroxy-5-pyrimidinyl)-4-chloro-3-cyanobenzamide

In a nitrogen atmosphere, 4-chloro-3-cyanobenzoic anhydride (1.00 g, 6.78 mmol) and 5-amino-4-hydroxypyrimidine (4.68 g, 13.6 mmol) were stirred under heating at 160°C for 1.5 hours, and cooled to room temperature. After addition of chloroform (100 mL), the crystalline precipitate was collected by filtration. The crystalline product was washed with chloroform (20 mL) and dried in air, to obtain 1.29 g (yield 69%) of the titled compound as a brown crystalline product.
m.p.: 255-260°C (decomp.)
$^1$H-NMR (DMSO-$d_6$, 400 MHz) δ: 5.5-6.5 (1H, br), 7.89 (1H, d, J=8Hz), 8.1-8.3 (1H, m), 8.3-8.4 (1H, m), 8.50 (1H, s), 8.64 (1H, s), 9.96 (1H, s).
FAB-MS (m/e): 275 (M+1)

(2) 2-(4-Chloro-3-cyanophenyl)oxazolo[5,4-d]pyrimidine

A solution of the above-mentioned N-(4-hydroxy-5-pyrimidinyl)-4-chloro-3-cyanobenzamide (100 mg, 0.36

mmol) in phosphoryl chloride (1 mL) was heated under reflux for 3 hours, cooled to room temperature, and placed under reduced pressure to distill phosphoryl chloride off. The reside was stirred for 30 minutes after addition of ice-water (2 mL). The crystalline precipitate was collected by filtration and recrystallized from ethanol (2 mL), to obtain 33 mg (yield 35%) of the titled compound as a brown crystalline product. A mixture of the mother liquor and the washing was placed under reduced pressure to distill the solvent off. The residue was purified by silica gel column chromatography (n-hexane/ethyl acetate), to obtain 15 mg (yield 16%) of the titled compound as a white crystalline product. Total yield: 51%.
$^1$H-NMR (DMSO-$d_6$, 400 MHz) δ: 8.04 (1H, d, J=8Hz), 8.53 (1H, d, J=8Hz), 8.79 (1H, s), 9.11 (1H, s), 9.40 (1H, s).

(3) 2-[3-Cyano-4-(4-fluorophenoxy)phenyl]oxazolo[5,4-d]-pyrimidine

To a suspension of 55% sodium hydride (20.4 mg, 0.47 mmol) in dry DMSO (1.5 mL) was added 4-fluorophenol (52.4 mg, 0.47 mmol). The mixture was stirred for 30 minutes at room temperature. After addition of the above-mentioned 2-(4-chloro-3-cyanophenyl)oxazolo[5,4-d]pyrimidine (40 mg, 0.16 mmol), the mixture was stirred at 50°C for 16 hours and cooled to room temperature. To the reaction mixture was added water (5 mL). The crystalline precipitate was collected by filtration, washed with water (20 mL), dried in air, purified by silica gel column chromatography (chloroform), and dried, to obtain 20 mg (yield 39%) of the titled compound as a white amorphous product.
$^1$H-NMR ($CDCl_3$, 400 MHz) δ: 7.07 (1H, d, J=9Hz), 7.3-7.4 (4H, m), 8.45 (1H, dd, J=2Hz, 9Hz), 8.70 (1H, d, J=2Hz), 9.07 (1H, s), 9.33 (1H, s).
IR (KBr) $cm^{-1}$: 2235, 1621, 1504, 1479, 1389, 1269, 1228, 1186, 1055, 1012, 914, 906, 847, 814, 793, 733, 698, 613, 503.
FAB-MS (m/e): 333 (M+1)

[Example 19] 2-[3-Cyano-4-(3-fluorophenylthio)phenyl]-thiazolo[5,4-d]pyrimidine

To a suspension of 55% sodium hydride (48 mg, 1.10 mmol) in dry DMSO (3 mL) was added 3-fluorothiophenol (141 mg, 1.10 mmol). The mixture was stirred for 30 minutes at room temperature. After addition of the above-mentioned 2-[4-chloro-3-cyanophenyl]thiazolo[5,4-d]pyrimidine (100 mg, 0.37 mmol), the mixture was stirred at 50°C for 3 hours and cooled to room temperature. To the reaction mixture was added water (5 mL). The crystalline precipitate was collected by filtration and purified by silica gel column chromatography (chloroform), and dried, to obtain 101 mg (yield 76%) of the titled compound as a white crystalline product.
m.p.: 189-190°C
$^1$H-NMR (DMSO-$d_6$, 400 MHz) δ: 7.3-7.6 (5H, m), 8.35 (1H, dd, J=2Hz, 8Hz), 8.64 (1H, d, J=2Hz), 9.19 (1H, s), 9.52 (1H, s).
IR (KBr) $cm^{-1}$: 2225, 1597, 1473, 1460, 1387, 1213, 1207, 1057, 1001, 876, 829, 789, 760, 690, 474.
FAB-MS (m/e): 365 (M+1)

[Example 20]

(1) N-(2-Hydroxyphenyl)acetamide

To a solution of 2-aminophenol (1.0 g, 9.16 mmol) in dry pyridine (50 mL) was added trimethylsilyl chloride (1.16 mL, 9.16 mmol). The mixture was stirred for one hour at room temperature. After addition of acetyl chloride (0.65 mL, 9.16 mmol) under ice-cooling, the mixture was stirred for 16 hours at room temperature. To the mixture was added aqueous ammonia (10 mL). The mixture was then stirred for 20 minutes at room temperature, placed under reduced pressure to degas ammonia, and subjected to extraction with ethyl acetate. The ethyl acetate extract was dried over anhydrous sodium sulfate, and the solvent was distilled off. The residue was crystallized from ethyl acetate, washed successively with n-hexane/ethyl acetate (1/1) and n-hexane, and dried in air, to obtain 976 mg (yield 70%) of the titled compound as a pale brown crystalline product.
$^1$H-NMR ($CDCl_3$, 400 MHz) δ: 2.27 (3H, s), 6.8-7.2 (4H, m), 7.26 (1H, s), 7.48 (1H, br s), 8.65 (1H, s).

(2) 2-[4-(2-Aminophenoxy)-3-cyanophenyl]thiazolo[5,4-d]-pyrimidine

To a suspension of 55% sodium hydride (48 mg, 1.10 mmol) in dry DMSO (3 mL) was added the above-mentioned N-(2-hydroxyphenyl)acetamide (166 mg, 1.10 mmol). The mixture was stirred for 20 minutes at room temperature. After addition of the aforementioned 2-(4-chloro-3-cyanophenyl)thiazolo[5,4-d]pyrimidine (100 mg, 0.37 mmol), the mixture was stirred at 50°C for 16 hours and then cooled to room temperature. To the reaction mixture was added water (5 mL). The aqueous mixture was subjected to extraction with chloroform. The chloroform extract was dried over anhydrous sodium sulfate, and the solvent was distilled off. The residue was purified by silica gel column chromatography (chloroform), to obtain 14 mg (yield 11%) of the titled compound as a yellow crystalline product.
m.p.: 256-259°C (decomp.)

$^1$H-NMR (DMSO-$d_6$, 400 MHz) δ: 6.73 (1H, d, J=9Hz), 6.86 (1H, t, J=7Hz), 6.96 (1H, d, J=9Hz), 7.12 (1H, t, J=7Hz), 7.21 (1H, d, J=7Hz), 8.13 (1H, d, J=9Hz), 8.31 (1H, m), 8.50 (1H, s), 9.09 (1H, s), 9.38 (1H, s), 9.75 (1H, s).
IR (KBr) $cm^{-1}$: 2345, 1460, 1117, 735.
FAB-MS (m/e): 346 (M+1)

[Example 21] 2-[3-Cyano-4-(3-pyridyloxy)phenyl]thiazolo-[5,4-d]pyrimidine hydrochloride

To a solution of the aforementioned 2-[3-cyano-4-(3-pyridyloxy)phenyl]thiazolo[5,4-d]pyrimidine (20 mg, 0.06 mmol) in chloroform (4 mL)-methanol (2 mL) was added several drops of hydrogen chloride-methanol. The mixture was placed under reduced pressure at a temperature of not higher than 30°C to distill the solvent off. The residue was concentrated under reduced pressure by distilling chloroform off, to obtain 22 mg (yield 100%) of the titled compound as a yellowish crystalline product.
m.p.: 204-206°C
$^1$H-NMR (DMSO-$d_6$, 400 MHz) δ: 7.20 (1H, d, J=9Hz), 7.6-7.7 (1H, m), 7.8-7.9 (1H, m), 8.44 (1H, dd, J=2Hz, 9Hz), 8.6-8.7 (2H, m), 8.72 (1H, d, J=2Hz), 9.63 (1H, s), 9.74 (1H, s).
IR (KBr) $cm^{-1}$: 2239, 1608, 1560, 1552, 1498, 1473, 1396, 1375, 1279, 1252, 1105.
FAB-MS (m/e): 332 (M+1)

[Example 22]

(1) 2-[3-Cyano-4-(4-tert-butoxypheoxy)phenyl]thiazolo-[5,4-d]pyrimidine

To a suspension of 55% sodium hydride (58 mg, 1.32 mmol) in dry DMSO (3 mL) was added 4-tert-butoxyphenol (219 mg, 1.32 mmol). The mixture was stirred at 50°C for 30 minutes. To the mixture was added the aforementioned 2-(4-chloro-3-cyanophenyl)thiazolo[5,4-d]pyrimidine (300 mg, 1.10 mmol). The mixture was then stirred at 50°C for 24 hours and cooled to room temperature. To the mixture was added water (15 mL). The crystalline precipitate was collected by filtration, washed successively with water (5 mL), ethanol (2 mL) and ether (5 mL), and dried under reduced pressure at room temperature, to obtain 342 mg (yield 77%) of the titled compound as a pale brown crystalline product.
$^1$H-NMR (DMSO-$d_6$, 400 MHz) δ: 1.33 (9H, s), 7.06 (1H, d, J=9Hz), 7.11 (2H, d, J=9Hz), 7.23 (2H, d, J=9Hz), 8.41 (1H, dd, J=2Hz, 9Hz), 8.66 (1H, d, J=2Hz), 9.18 (1H, s), 9.51 (1H, s).

(2) 2-[3-Cyano-4-(4-hydroxyphenoxy)phenyl]thiazolo[5,4-d]pyrimidine

A mixture of the above-mentioned 2-[3-cyano-4-(4-tert-butoxypheoxy)phenyl]thiazolo[5,4-d]pyrimidine [5,4-d]pyrimidine (340 mg, 0.84 mmol) and trifluoroacetic acid (3 mL) was stirred for 2 hours at room temperature and placed under reduced pressure to distill the solvent off. The residue was stirred for 2 hours at room temperature after addition of ethanol (5 mL). The resulting crystalline product was collected by filtration, washed successively with ethanol (5 mL) and ether (5 mL), and dried under reduced pressure at room temperature, to obtain 261 mg (yield 89%) of the titled compound as a pale yellow crystalline product.
m.p.: >250°C
$^1$H-NMR (DMSO-$d_6$, 400 MHz) δ: 6.88 (2H, d, J=9Hz), 6.97 (1H, d, J=9Hz), 7.11 (2H, d, J=9Hz), 8.37 (1H, dd, J=2Hz, 9Hz), 8.62 (1H, d, J=2Hz), 9.16 (1H, s), 9.48 (1H, s), 9.62 (1H, s).
IR (KBr) $cm^{-1}$: 2235, 1606, 1572, 1502, 1477, 1400, 1381, 1279, 1257, 1236, 1194, 1105, 993, 847, 835, 796, 758, 729, 687, 648, 619, 598, 532, 494, 463.
FAB-MS (m/e): 347 (M+1)

[Example 23]

(1) 2-[3-Cyano-4-(2-benzyloxycarbonylphenoxy)phenyl]-thiazolo[5,4-d]pyrimidine

To a suspension of 55% sodium hydride (53 mg, 2.20 mmol) in DMSO (5.0 mL) was added benzyl 2-hydroxybenzoate (503 mg, 2.20 mmol). The mixture was stirred for 30 minutes at room temperature. To the mixture was added the aforementioned 2-(4-chloro-3-cyanophenyl)thiazolo-[5,4-d]pyrimidine (200 mg, 0.73 mmol). The mixture was then stirred at 50°C for 40 hours and cooled to room temperature. To the mixture was added water (10 mL). The gum-like precipitate was collected by filtration, washed with water (5 mL x 2), and dissolved in ethyl acetate. The ethyl acetate solution was placed under reduced pressure to distill the solvent off. The residue was mixed with ethanol (5 mL), heated under reflux for 20 minutes, and cooled to room temperature. The crystalline product was collected by filtration, washed with ethanol (2 mL), dried in air, and the dried under reduced pressure at room temperature, to obtain 254 mg (yield

74%) of the titled compound as a pale yellow crystalline product.
$^1$H-NMR ($CDCl_3$, 400 MHz) δ: 5.19 (2H, s), 6.62 (1H, d, J=9Hz), 7.1-7.3 (6H, m), 7.45 (1H, td, J=8Hz, J=1Hz), 7.68 (1H, td, J=4Hz, J=8Hz), 8.01 (1H, dd, J=9Hz, 2Hz), 8.1-8.2 (2H, m), 9.14 (1H, s), 9.35 (1H, s).

(2) 2-[3-Cyano-4-(2-hydroxycarbonylphenoxy)phenyl]-thiazolo[5,4-d]pyrimidine

To a solution of the above-mentioned 2-[3-cyano-4-(2-benzyloxycarbonylphenoxy)phenyl]thiazolo[5,4-d]pyrimidine (100 mg, 0.22 mmol) in THF (3 mL) was added 2M aqueous sodium hydroxide (0.5 mL). The mixture was stirred for 18 hours at room temperature, neutralized with 1N aqueous hydrochloric acid, and placed under reduced pressure to distill the solvent off. The residue was dissolved in chloroform, washed with 3 portions of water, dried over anhydrous sodium sulfate, and placed under reduced pressure to distill the solvent off. The residue was purified by silica gel column chromatography (chloroform/methanol) and then recrystallized from ethanol (1 mL), to obtain 11 mg (yield 14%) of the titled compound as a pale yellow crystalline product.
$^1$H-NMR (DMSO-$d_6$, 400 MHz) δ: 6.84 (1H, d, J=8Hz), 7.4-7.6 (2H, m), 7.78 (1H, dd, J=7Hz, 7Hz), 8.01 (1H, d, J=1Hz), 8.34 (1H, dd, J=2Hz, 9Hz), 8.66 (1H, d, J=2Hz), 9.17 (1H, s), 9.50 (1H, s).
FAB-MS (m/e): 375 (M+1)

[Example 24] 2-[3-Cyano-4-(2-hydroxyphenoxy)phenyl]-thiazolo[5,4-d]pyrimidine potassium salt

2-[3-Cyano-4-(2-hydroxyphenoxy)phenyl]thiazolo[5,4-d]pyrimidine (Example 8, 28 mg, 0.08 mmol) was dissolved in a mixture of chloroform (5.6 mL) and methanol (5.6 mL). To the solution was added a solution of potassium hydroxide (6 mg, 0.09 mmol) in methanol (0.6 mL). Thus obtained yellow solution was concentrated under reduced pressure. The residue was concentrated under reduced pressure after addition of ethanol (3 mL). The residue was pulverized in hexane (1 mL), collected by filtration, and washed with hexane (3 mL), to obtain 28 mg (yield 91%) of the titled compound as a gray powder.
m.p.: not clear
$^1$H-NMR (DMSO-$d_6$, 400 MHz) δ: 6.84 (1H, d, J=9Hz), 6.3-7.2 (4H, m), 8.30 (1H, dd, J=2Hz, 9Hz), 8.53 (1H, d, J=2Hz), 9.14 (1H, s), 9.46 (1H, s).
IR (KBr) $cm^{-1}$: 2231, 1610, 1576, 1508, 1479, 1458, 1400, 1375, 1294, 1265, 1228, 1176, 1113, 1095, 833, 748.

[Example 25] 2-[3-Cyano-4-(4-hydroxyphenoxy)phenyl]-thiazolo[5,4-d]pyrimidine potassium salt

2-[3-Cyano-4-(4-hydroxyphenoxy)phenyl]thiazolo[5,4-d]pyrimidine (Example 22, 200 mg, 0.58 mmol) was dissolved in ethanol (4 mL). To the solution was added a solution of potassium hydroxide (0.5 mol/L) in ethanol (1.27 mL). The mixture was for one hour at room temperature and placed under reduced pressure to distill the solvent off. The residue was dispersed in ethanol (1 mL), and hexane (1 mL) was added. The crystalline precipitate was collected by filtration, washed successively with ethanol/hexane (1/2 mixture, 1.5 mL) and hexane (2 mL), and dried under reduced pressure at room temperature, to obtain 189 mg (yield 95.2%) of the titled compound as an orange crystalline product.
m.p.: >250°C (decomp.)
$^1$H-NMR (DMSO-$d_6$, 400 MHz) δ: 6.44 (2H, d, J=8Hz), 6.78 (2H, d, J=8Hz), 7.00 (1H, d, J=9Hz), 8.36 (1H, dd, J=2Hz, 9Hz), 8.57 (1H, d, J=2Hz), 9.15 (1H, s), 9.48 (1H, s).
IR (KBr) $cm^{-1}$: 3396, 3032, 2235, 1606, 1491, 1462, 1400, 1373, 1263, 1186, 1111, 1109, 847.

[Example 26] Pharmacological Experiment 1 (*in vitro* measuring method)

(Measuring procedure)

1. Preparation of test sample

The test compound was dissolved in dimethylsulfoxide and diluted with 50 mM phosphate buffer (pH 7.5), to give a solution of a predetermined concentration.

2. Measurement

125 μL of each of the solutions of the test compound having different concentrations was added to 1 mL of a solution of Xanthine (SIGMA, 250 μM) in the 50 mM phosphor buffer (pH 7.5). The mixture was then pre-incubated at 30°C for 5 min. Subsequently, to the pre-incubated mixture was added 125 μL of Cow milk Xanthine Oxidase (Roche) diluted with the 50 mM phosphate buffer (pH 7.5) to 70 mU/mL, and the mixture was subjected to reaction at 30°C for

10 minutes. Then, 1N hydrochloric acid (200 μL) was added to the reaction mixture to terminate the reaction. Subsequently, absorbance at OD 290 nm was measured by means of a spectrophotometer (Shimadzu UV-160A), to obtain a inhibition ratio. The measured inhibition ration was used to obtain $IC_{50}$.

The inhibition ratio was calculated according to the following formula:

$$\text{Inhibition ratio (\%)} = [1 - (B-C)/(A-C)] \times 100$$

A: absorbance of control
B: absorbance measured on a sample containing test compound
C: absorbance of blank

(Test results)

The test results are set forth in Tables 11 and 12.

As is apparent from Tables 11 and 12, the compounds of the present invention show an excellent xanthine oxidase inhibiting action in *in vitro* pharmacological test.

[Example 27] Pharmacological experiment 2 (*in vivo* test)

(Test method)

The test compound suspended in 1% methylcellulose solution in an amount of 0.3 mg/kg or 3 mg/kg was administered to unfasted ICR mouse (7 W) by forced single oral administration. The blood was collected from main artery from the mouse under etherization after one hour from the administration. The plasma was separated from the collected blood in the conventional manner. The plasma was then subjected to measurement of uric acid value by the enzyme method by means of an automatic analytical apparatus (7060E), to obtain an in-plasma uric acid value-inhibition ratio in the test sample-administered group as compared with a ratio obtained in the normal group.

Based on the obtained in-plasma uric acid inhibition ratio, activity values (%) relative to activity values obtained in the simultaneously conducted tests using TMX-67 (0.3 mg/kg).

(Test results)

The test results are set forth in Tables 11 and 12.

As is apparent from Tables 11 and 12, the compounds of the present invention show an excellent xanthine oxidase inhibiting action in *in* vivo pharmacological test.

Table 11

| Test compound (Example No.) | In Vitro $IC_{50}$ (nM) | In Vivo(%) TMX-67 (0.3 mg/kg) |
|---:|---:|---:|
| Example 1 | 280 | 132 |
| 2 | 139 | 114 |
| 3 | 273 | 90 |
| 4 | 33 | 33 |
| 5 | 103 | 141 |
| 6 | 389 | 127 |
| 7 | 239 | 109 |
| 8 | 103 | 52 |
| 9 | 298 | 120 |
| 10 | 127 | 115 |
| 11 | 196 | 110 |

The Example No. corresponds to the aforementioned Example.

Table 12

| Test compound (Example No.) | In Vitro $IC_{50}$ (nM) | In Vivo (%) TMX-67 (0.3 mg/kg) |
|---|---|---|
| Example 12 | 310 | 52 |
| 13 | | 40 |
| 14 | | 43 |
| 15 | | 87 |
| 16 | | 34 |
| 17 | | 86 |
| 18 | | 71 |
| 19 | | 171 |
| 20 | 58 | |
| 21 | | 68 |
| 22 | 72 | |
| 25 | | 35 |
| 26 | | |

The Example No. corresponds to the aforementioned Example.

## Claims

**1.** Compounds of the following formula (I) or salts thereof:

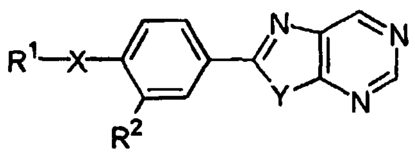

(I)

in which

$R^1$ represents an alkenyl group having 2-8 carbon atoms, or an aryl group having 6-10 carbon atoms or a heteroaryl group which may have a substituent selected from the group and atom consisting of an alkyl group having 1-8 carbon atoms, a halogen-substituted alkyl group having 1-8 carbon atoms, an alkoxy group having 1-8 carbon atoms, a halogen-substituted alkoxy group having 1-8 carbon atoms, an alkoxycarbonyl group having 2-8 carbon atoms, formyl, carboxyl, a halogen atom, hydroxyl, nitro, cyano, amino, an aryl group having 6-10 carbon atoms, and an aryloxy group having 6-10 carbon atoms;
$R^2$ represents cyano, nitro, formyl, carboxyl, carbamoyl, or an alkoxycarbonyl group having 2-8 carbon atoms;
X represents oxygen, $-N(R^3)-$ or $-S(O)_n-$ in which $R^3$ is hydrogen, an alkyl group having 1-8 carbon atoms or the above-mentioned group described for $R^1$, or $R^3$ is combined with $R^1$ to form morpholinyl, thiomorpholinyl or piperazinyl, and n is an integer of 0 to 2; and
Y represents oxygen, sulfur or NH.

**2.** The compounds or salts thereof defined in claim 1, in which $R^1$ represents a phenyl, naphthyl, furyl, pyrrolyl, thienyl, piperidinyl, pyrimidinyl, pyranyl, pyridyl, thiazolyl, imidazolyl, indolyl or quinolyl group which may have a substituent selected from the group and atom consisting of an alkyl group having 1-8 carbon atoms, a halogen-substituted alkyl group having 1-8 carbon atoms, an alkoxy group having 1-8 carbon atoms, a halogen-substituted alkoxy group having 1-8 carbon atoms, an alkoxycarbonyl group having 2-8 carbon atoms, formyl, carboxyl, a halogen atom, hydroxyl, nitro, cyano, amino, an aryl group having 6-10 carbon atoms, and an aryloxy group having 6-10 carbon atoms.

3. The compounds or salts thereof defined in claim 1, in which $R^1$ represents a phenyl or pyridyl group which may have a substituent selected from the group and atom consisting of an alkyl group having 1-8 carbon atoms, a halogen-substituted alkyl group having 1-8 carbon atoms, an alkoxy group having 1-8 carbon atoms, a halogen-substituted alkoxy group having 1-8 carbon atoms, an alkoxycarbonyl group having 2-8 carbon atoms, formyl, carboxyl, a halogen atom, hydroxyl, nitro, cyano, amino, an aryl group having 6-10 carbon atoms, and an aryloxy group having 6-10 carbon atoms.

4. The compounds or salts thereof defined in claim 1, in which $R^1$ represents a phenyl or pyridyl group which may have a substituent selected from the group and atom consisting of an alkyl group having 1-8 carbon atoms, a halogen-substituted alkyl group having 1-8 carbon atoms, an alkoxy group having 1-8 carbon atoms, carboxyl, a halogen atom, hydroxyl, nitro, cyano, and amino.

5. The compounds or salts thereof defined in any one of claims 1 to 4, in which $R^2$ represents cyano or nitro.

6. The compounds or salts thereof defined in any one of claims 1 to 4, in which $R^2$ represents cyano.

7. The compounds or salts thereof defined in any one of claims 1 to 6, in which X is oxygen, NH or sulfur.

8. The compounds or salts thereof defined in any one of claims 1 to 6, in which X is oxygen or sulfur.

9. The compounds or salts thereof defined in any one of claims 1 to 8, in which Y is sulfur or NH.

10. The compounds or salts thereof defined in any one of claims 1 to 8, in which Y is sulfur.

11. The compounds having the formula (I) or salts thereof defined in claim 1, in which $R^1$ represents a phenyl or pyridyl group which may have a substituent selected from the group and atom consisting of an alkyl group having 1-8 carbon atoms, a halogen-substituted alkyl group having 1-8 carbon atoms, an alkoxy group having 1-8 carbon atoms, carboxyl, a halogen atom, hydroxyl, nitro, cyano, and amino; $R^2$ represents cyano or nitro; X is oxygen or sulfur; and Y is sulfur or NH.

12. The compounds having the formula (I) or salts thereof defined in claim 1, in which $R^1$ represents a phenyl or pyridyl group which may have a substituent selected from the group and atom consisting of an alkyl group having 1-8 carbon atoms, a halogen-substituted alkyl group having 1-8 carbon atoms, an alkoxy group having 1-8 carbon atoms, carboxyl, a halogen atom, hydroxyl, nitro, cyano, and amino; $R^2$ represents cyano or nitro; X is oxygen or sulfur; and Y is sulfur.

13. The compounds having the formula (I) or salts thereof defined in claim 1, in which $R^1$ represents a phenyl or pyridyl group which may have a substituent selected from the group and atom consisting of an alkyl group having 1-8 carbon atoms, a halogen-substituted alkyl group having 1-8 carbon atoms, an alkoxy group having 1-8 carbon atoms, carboxyl, a halogen atom, hydroxyl, nitro, cyano, and amino; $R^2$ represents cyano or nitro; X is oxygen; and Y is sulfur.

14. A xanthine oxidase inhibitor containing as an active ingredient a compound or a salt thereof according to any one of claims 1 to 13.

15. An agent for treating hyperuricemia containing as an active ingredient a compound or a salt thereof according to any one of claims 1 to 13.

**INTERNATIONAL SEARCH REPORT**

International application No. PCT/JP2006/313443

A. CLASSIFICATION OF SUBJECT MATTER
*C07D473/00*(2006.01)i, *A61K31/519*(2006.01)i, *A61K31/52*(2006.01)i, *A61K31/5377*(2006.01)i, *A61K31/541*(2006.01)i, *A61P19/06*(2006.01)i, *A61P43/00*(2006.01)i, *C07D498/04*(2006.01)i, *C07D513/04*(2006.01)i

According to International Patent Classification (IPC) or to both national classification and IPC

B. FIELDS SEARCHED

Minimum documentation searched (classification system followed by classification symbols)
C07D473/00, A61K31/519, A61K31/52, A61K31/5377, A61K31/541, A61P19/06, A61P43/00, C07D498/04, C07D513/04

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2006 |
| Kokai Jitsuyo Shinan Koho | 1971-2006 | Toroku Jitsuyo Shinan Koho | 1994-2006 |

Electronic data base consulted during the international search (name of data base and, where practicable, search terms used)
CAOLD(STN), CAplus(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| X | WO 03/042185 A1 (Nippon Chemiphar Co., Ltd.), 22 May, 2003 (22.05.03), & EP 1452528 A1 & US 2005/090472 A1 & CN 1615299 A | 1-15 |
| P,A | WO 2005/121153 A1 (Nippon Chemiphar Co., Ltd.), 22 December, 2005 (22.12.05), (Family: none) | 1-15 |
| A | JP 3-163082 A (Otsuka Pharmaceutical Factory, Inc.), 15 July, 1991 (15.07.91), & US 5137887 A & EP 414200 A2 & AU 6098590 A & CN 1049664 A & KR 9602851 B & CA 2023430 A | 1-15 |

☐ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 29 August, 2006 (29.08.06) | 05 September, 2006 (05.09.06) |

| Name and mailing address of the ISA/ | Authorized officer |
|---|---|
| Japanese Patent Office | |
| Facsimile No. | Telephone No. |

Form PCT/ISA/210 (second sheet) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9209279 A
- WO 9818765 A
- JP 121431 A
- WO 03064410 A
- WO 03042185 A